(19) Europäisches Patentamt
European Patent Office
Office européen des brevets

(11) Veröffentlichungsnummer: **0 315 831 B1**

(12) **EUROPÄISCHE PATENTSCHRIFT**

(45) Veröffentlichungstag der Patentschrift: **30.12.92**

(51) Int. Cl.[5]: **C12N 11/08**

(21) Anmeldenummer: **88117889.1**

(22) Anmeldetag: **27.10.88**

# (54) Verfahren zur Herstellung eines Biokatalysators und dessen Verwendung zur Razematspaltung.

(30) Priorität: **04.11.87 DE 3737335**

(43) Veröffentlichungstag der Anmeldung:
**17.05.89 Patentblatt 89/20**

(45) Bekanntmachung des Hinweises auf die Patenterteilung:
**30.12.92 Patentblatt 92/53**

(84) Benannte Vertragsstaaten:
**DE ES FR GB IT NL SE**

(56) Entgegenhaltungen:
**EP-A- 0 075 815**
**EP-A- 0 088 964**
**EP-A- 0 150 350**
**EP-A- 0 210 499**
**US-A- 4 013 825**

**APPLIED BIOCHEMISTRY & MICROBIOLOGY, Band 14, Nr. 5, September/Oktober, 1978, Seiten 544-548, Plenum Publishing Corp., New York, US; M.Kh. TADZHIEV et al.: "Immobilization and stabilization of enzymes by polymers with functional epoxy groups"**

(73) Patentinhaber: **BASF Aktiengesellschaft**
**Carl-Bosch-Strasse 38**
**W-6700 Ludwigshafen(DE)**

(72) Erfinder: **Urban, Dieter, Dr.**
**Espenstrasse 15**
**W-6800 Mannheim 1(DE)**
Erfinder: **Ladner, Wolfgang, Dr.**
**In den Bellen 5**
**W-6701 Fussgoenheim(DE)**
Erfinder: **Paul, Axel, Dr.**
**Kaefertaler Strasse 38**
**W-6800 Mannheim 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein einfaches und wirtschaftliches Verfahren zur Herstellung eines Immobilisates von roher Pankreaslipase auf Basis eines Polymerisats mindestens eines Vinyllactams, das Immobilisat selbst und dessen Verwendung zur Razematspaltung von Estern razemischer Alkohole.

Techniken zur Immobilisierung von Lipase sind bekannt. Gemäß US 4 629 742 wird zur Immobilisierung Lipase aus Candida cylindracea eingesetzt. Als Basis für das Immobilisat bewahrten sich hydrophobe Polymere, vor allem Polyethylen und Polypropylen.

Das in der EP-A-210 499 u.a. auch für Pankreaslipase beschriebene Immobilisierungsverfahren durch Gelbildung aus Enzym, Polyepoxid und Polyamin ist zwar einfach, führt jedoch zu Biokatalysatoren, die infolge ihrer geringen mechanischen Stabilität und schlechter hydrodynamischer Eigenschaften (Filtrierbarkeit; Strömungswiderstand in damit gepackten Säulen) für den technischen Gebrauch wenig geeignet sind.

Auch die enantioselektive Spaltung razemischer Ester durch Schweinepankreaslipase im Labormaßstab ist bekannt (W.E. Ladner und G.M. Whitesides, JACS 1984, 106, 7250 - 7251). Dabei wurde rohe, nicht immobilisierte Lipase eingesetzt. Dieses Verfahren ist zur technischen Durchführung ungeeignet, ein wirtschaftliches Verfahren erfordert eine Immobilisierung. Eine solche nach üblichen Methoden, etwa nach dem obengenannten US-Patent, führte nicht zum Ziel, denn die spezifische Aktivität des Immobilisats war niedrig und ließ im Betrieb viel zu rasch nach.

Der Erfindung lag daher die Aufgabe zugrunde, Immobilisate roher Pankreaslipase leicht und preiswert herzustellen, so daß ein einfaches, wirtschaftliches und für den technischen Maßstab geeignetes Verfahren zur Razematspaltung von Estern optisch aktiver Alkohole durchführbar ist. Wichtige Kriterien eines geeigneten Biokatalysators sind lange Lebensdauer und hohe Raum-Zeit-Ausbeuten.

Die Lösung der Aufgabe besteht in dem Verfahren nach den Ansprüchen 1 bis 9. Die Herstellung des Biokatalysators erfolgt durch Mischen eines pulvrigen, unlöslichen, nur wenig quellbaren Copolymerisates mindestens eines N-Vinyllactams mit 4 bis 6 Kohlenstoffatomen, vorzugsweise N-Vinylpyrrolidon, mit 0,5 bis 10 Gew.%, bezogen auf die gesamten Monomeren, eines cyclischen Säureamids, das mindestens 2 ethylenisch ungesättigte, copolymerisierbare Gruppen enthält, von denen mindestens eine unmittelbar an Amidstickstoff gebunden ist, vorzugsweise N,N' -Divinylethylenharnstoff, einer Lipase und Wasser bei pH 5 bis 9, vorzugsweise 5,5 bis 8 und 0 bis 40, vorzugsweise 10 bis 30 °C, nach einer Minute bis 3 Tagen, vorzugsweise ein bis zehn Stunden, Filtrieren und Waschen des Filterkuchens mit Wasser oder Pufferlösung oder einem wasserlöslichen organischen Lösungsmittel, wobei als Lipase rohe, d.h. bis zu 99 Gew.% natürliche Verunreinigungen (vor allem Proteine) enthaltende und damit sehr leicht und preiswert zugängliche, handelsübliche Schweinepankreaslipase eingesetzt wird, und wobei der wäßrigen Mischung als Stabilisator für die Lipase (zur zeitlichen Verlängerung ihrer Aktivität) mindestens ein wasserlöslicher aliphatischer Alkohol mit zwei bis sechs Kohlenstoffatomen und zwei bis sechs Hydroxylgruppen, gegebenenfalls im Gemisch mit einem einwertigen Alkohol mit 1 bis 4 Kohlenstoffatomen, zugesetzt wird.

Die Herstellung des erfindungsgemäß einzusetzenden Copolymerisates eines oder mehrer Vinyllactame mit einem cyclischen Säureamid, das mindestens 2 ethylenisch ungesättigte, copolymerisierbare Gruppen enthält, von denen mindestens eine an Amidstickstoff gebunden ist, kann nach US 3 992 562 oder US 4 013 825 erfolgen. Die Polymerisation verläuft ohne die üblichen Starter spontan unter Luftausschluß bei pH 6 bis 9 und erhöhter Temperatur, und zwar entweder in Gegenwart geringer Mengen einer Schwefelverbindung, in der der Schwefel eine niedrigere Wertigkeit als 6 aufweist, oder in Gegenwart einer $\alpha$- oder $\beta$-Ketocarbonsäure. Die Copolymerisate sind von Natur aus porös.

Geeignete Vinyllactame, die allein oder in Mischung miteinander mit den als Vernetzer wirkenden, mindestens 2 copolymerisierbare Doppelbindungen enthaltenden Amiden copolymerisiert werden können, sind N-Vinylcaprolactam, N-Vinylpiperid-2-on und vorzugsweise N-Vinylpyrrolidon.

Als cyclische Amide mit mindestens zwei ethylenisch ungesättigten, copolymerisierbaren Doppelbindungen wird N,N'-Divinylethylenharnstoff bevorzugt.

Die Korngröße des Copolymerisates soll entsprechend dem jeweiligen Einsatz im Bereich von 1 µm bis 1 mm, vorzugsweise 10 bis 500 µm liegen. Die Untergrenze wird durch die Filtrierbarkeit bzw. den hydrodynamischen Widerstand einer damit gepackten Säule bestimmt, die Obergrenze durch die Diffusionszeit für die Substratlösung in das Teilcheninnere (die wiederum u.a. von der Porengröße abhängt). Die Porengröße liegt im Bereich von 2 bis 200, vorzugsweise 5 bis 100 nm, gemessen durch Quecksilber-Porosimetrie.

Die Lipase selbst ist zwar wasserlöslich, doch sind viele Bestandteile der rohen Lipase wasserunlöslich und werden im Reaktionsgemisch suspendiert.

Mit wasserlöslichen Lösungsmitteln und Alkoholen sind solche gemeint, die bei 20 °C zu mindestens

10, besser zu mindestens 20 Gew.% in Wasser löslich sind. Besonders bevorzugt sind Lösungsmittel und Alkohole, die in jedem Verhältnis mit Wasser mischbar sind. Mit der "Wertigkeit" des Alkohols ist die Zahl der Hydroxylgruppen gemeint. Mit "nur wenig quellbar" ist eine Wasseraufnahmefähigkeit in der Größenordnung von 1 bis 3 Gew.% gemeint.

Als wasserlösliche Alkohole, die zur zeitlichen Verlängerung der Aktivität der Lipase eingesetzt werden, kommen beispielsweise in Betracht:

Ethylenglykol, 1,2- und 1,3-Propandiol, 1,2-, 1,3-, 1,4- und 2,3-Butandiol, 1,2- und 1,3-i-Butandiol, 1,2-und 1,5-Pentandiol, Diethylenglykol, Glyzerin, Erythrit, Pentaerythrit, Arabit, Xylit, Sorbit, Mannit und Dulcit und deren Mischungen. Bevorzugt werden mehrwertige wasserlösliche Alkohole mit 2 bis 6 OH-Gruppen allein oder in Mischungen verschiedenwertiger Alkohole, insbesondere Glyzerin und Mischungen aus Glyzerin mit Methanol, Ethanol, 2-Propanol und 1,5-Pentandiol. Ein Teil dieser mehrwertigen Alkohole kann auch durch einen oder mehrere folgender einwertiger Alkohole ersetzt sein: Methanol, Ethanol, 1- und 2-Propanol, 2-Butanol und t-Butanol.

Zur Einstellung des pH-Wertes im Bereich von 5 bis 9, vorzugsweise 5,5 bis 8, verwendet man zweckmäßig die üblichen Puffermischungen, beispielsweise Mischungen von primären und sekundären Phosphaten, Borax, Mischungen von Tris-(hydroxymethyl)amin und dessen Hydrochlorid, Acetatpuffermischungen.

Die Gesamtmischung setzt sich folgendermaßen zusammen:

1 bis 50, vorzugsweise 4 bis 30 % des beschriebenen Copolymerisates von N-Vinyllactam,

1 bis 30, vorzugsweise 5 bis 25 % rohe Schweinepankreaslipase,

1 bis 68, vorzugsweise 20 bis 60 % mindestens eines der beschriebenen wasserlöslichen, mehrwertigen Alkohole, wobei die über 1, vorzugsweise über 5, insbesondere 10 % hinausgehende Menge durch einen wasserlöslichen einwertigen Alkohol mit 1 bis 4 C-Atomen ersetzt sein kann,

30 bis 97 % Wasser

sowie den pH-Wert regulierende Zusätze (die gewichtsmäßig kaum eine Rolle spielen). Die Prozente beziehen sich jeweils auf das Gewicht der Gesamtmischung.

Die Komponenten können in beliebiger Reihenfolge gemischt werden, vorzugsweise suspendiert man die rohe Lipase in der Mischung aus Wasser und Alkohol und gibt danach den polymeren Träger zu.

Die Mischung läßt man zweckmäßig eine Minute bis 3 Tage, vorzugsweise 1 Stunde bis 1 Tag bei 0 bis 40, vorzugsweise 10 bis 30 ° C verweilen, wobei man sie vorzugsweise in Bewegung hält (z. B. Rühren, Rollen). Dann wird filtriert. Der Filterkuchen kann unmittelbar, gewaschen oder ungewaschen, in wasserhaltigem Medium zur Esterspaltung eingesetzt werden. Vorteilhaft wird er aber vorher mit einer wäßrigen oder wäßrig-alkoholischen Lösung eines Vernetzungsmittels zur Fixierung der Lipase eine Minute bis 2 Stunden, vorzugsweise 2 Minuten bis 1 Stunde lang bei Raumtemperatur (10 bis 30 ° C behandelt, d.h. in Kontakt gebracht. Geeignete Vernetzungsmittel sind z.B. Dialdehyde, insbesondere Glutardialdehyd und Glyoxal, sowie wasserlösliche Glycidylether, hergestellt gemäß EP-A 210 499, durch Anlagerung von 1 bis 30 Äquivalenten eines Epoxids der Formel I

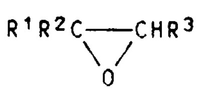

I,

worin die Reste $R^1$, $R^2$ und $R^3$ Wasserstoffatome oder Methyl- oder Ethylgruppen darstellen, an je 1 Äquivalent (bezogen auf OH-Gruppen) eines polyfunktionellen Alkohols mit 2 bis 6 Kohlenstoffatomen oder eines Mono- oder Disaccharids und Umsetzung des so erhaltenen Anlagerungsproduktes mit pro OH-Gruppe einem Äquivalent Epichlorhydrin und anschließende Cyclisierung zum Epoxid.

Die Verwendung des Immobilisates kann in üblicher Weise, z.B. in Säulen oder im Rührreaktor, erfolgen.

Durch das erfindungsgemäße Verfahren wird zum ersten Mal Schweinepankreaslipase in ein wiederverwendbares, gut filtrierbares, für den Einsatz in Säulen geeignetes Immobilisat von hoher spezifischer Aktivität und langer Einsatzdauer überführt. Die Tatsache, daß Nebenbestandteile roher Lipase dabei nicht stören, macht das Verfahren besonders wirtschaftlich.

Bei den für die Beispiele verwendeten Copolymerisaten aus N-Vinvlpyrrolidon und N,N' -Divinylethylenharnstoff hatten Poren mit einem Durchmesser von 10 bis 50 nm die größte Häufigkeit. Die Korngröße lag immer unter 500 $\mu$m.

Bestimmung der enzymatischen Aktivität von Pankreatin

In einer pH-Stat-Apparatur werden 50 ml Borax/HCl-Puffer (20 mM, pH 7,5) und 1 ml Glycidylbutyrat miteinander gemischt und bei 30 °C intensiv gerührt. Nach Zugabe einer geeigneten Menge (üblicherweise 1-10 mg) roher Pankreaslipase bzw. des zu testenden Immobilisats (500 mg) wird durch automatische Dosierung von 0,5 N NaOH-Lösung der pH konstant gehalten. Die spezifische enzymatische Aktivität $A_{sp}$ wird wie folgt berechnet:

$$A_{sp} = \frac{\Delta V \cdot N}{\Delta t \cdot m} .$$

Dabei bedeuten:
$\Delta V/\Delta t$ den Verbrauch der Natronlauge zu Beginn der Reaktion in ml/min, N die Normalität der Natronlauge (500 µmol/ml) und m die Einwaage des Enzyms bzw. des Immobilisates.

Die übliche Einheit für die enzymatische Aktivität ist 1 unit (Abkürzung: u), das entspricht einem NaOH-Verbrauch von 1 µmol/min.

Die spezifische Aktivität des zur Immobilisierung verwendeten Pankreatins der Fa. Nordmark betrug 17 u/mg.

Die in den Beispielen genannten Teile und Prozente beziehen sich auf das Gewicht.

Herstellung des Immobilisates

Beispiel 1

In 18 g einer Mischung aus 80 Teilen 200 mmolarem Phosphatpuffer, pH 7,0, 10 Teilen Glycerin und 10 Teilen Isopropanol suspendierte man 2 g rohe Schweinepankreaslipase. Zu dieser Suspension gab man 1 g eines Copolymerisats, hergestellt nach US 3 992 562, aus 100 Teilen N-Vinylpyrrolidon und 2 Teilen N,N'-Divinylethylenharnstoff.

Diese Mischung blieb über Nacht bei Raumtemperatur unter Luftausschluß stehen und wurde am nächsten Tag mit 5,4 g 25 %iger wäßriger Glutardialdehydlösung versetzt. Nach einer Stunde wurde das Immobilisat abgesaugt und mit einer Mischung aus 80 Teilen 200 mmolarem Phosphatpuffer, 10 Teilen Glyzerin und 10 Teilen Isopropanol gewaschen.

Man erhielt 2,7 g Immobilisat. Dieses wurde zweimal zur Esterspaltung eingesetzt, die spezifischen Enzymaktivitäten stehen in der Tabelle:

| Ansatz | $A_{sp}$ [u/g] | $A_{rel.}$ in % |
|---|---|---|
| 1 | 1333 | - |
| 2 | 1282 | 96 |

Beispiel 2

In 15 g einer Mischung aus 70 Teilen 50 mmolarem Boratpuffer, pH 8,0, 20 Teilen Glyzerin und 10 Teilen Ethanol suspendierte man 1 g rohe Schweinepankreaslipase. Zu dieser Suspension gab man 1 g eines Copolymerisates, hergestellt nach US 3 992 562 aus 100 Teilen N-Vinylpyrrolidon und 2 Teilen N,N'-Divinylethylenharnstoff. Diese Mischung blieb über Nacht bei 8 °C unter Luftausschluß stehen und wurde am nächsten Tag mit 3,0 g Sorbit-$EO_{80}$-Epoxid versetzt. Nach einer Stunde saugte man das Immobilisat ab und wusch mit der obigen Mischung aus Boratpuffer, Glyzerin und Ethanol. Man erhielt 2,9 g Immobilisat. Dieses wurde zweimal zur Esterspaltung eingesetzt, die spezifischen Aktivitäten stehen in der Tabelle:

| Ansatz | $A_{sp}$ [u/g] | $A_{rel.}$ in % |
|---|---|---|
| 1 | 980 | - |
| 2 | 952 | 97 |

Das Sorbit-$EO_{80}$-Epoxid hatte einen Epoxidwert von 1,2 mmol/g und wurde wie folgt hergestellt (DE 35 27 014):
3705 g Sorbit-$EO_{80}$ (Reaktionsprodukt aus 1 Mol Sorbit mit 80 Mol Ethylenoxid, vgl. Houben-Weyl, Methoden der Org. Chemie 14/2, (1963), S. 450) werden mit 14,8 g $BF_3$-Dihydrat versetzt. Danach werden bei 70 ° C unter Rühren 555 g Epichlorhydrin zugetropft. Es wird weitere 2 Stunden bei 70 ° C gerührt und anschließend bei 20 bis 35 ° C 528 g 50 %ige Natronlauge im Verlauf von 1 bis 2 Stunden zugetropft. Es wird so lange weitergerührt, bis etwa 90 % der Natronlauge verbraucht sind. Der Natronlauge-Verbrauch wird titrimetrisch verfolgt.

Die Hauptmenge des Wassers wird bei 70 ° C im Wasserstrahlvakuum abdestilliert und der Rest heiß (70 ° C) abgesaugt.

So werden 3439 g (85 %) Umsetzungsprodukt von Epichlorhydrin mit Sorbit-$EO_{80}$ erhalten, welches einen Epoxid-Titer von 1,2 mmol/g besitzt.

Beispiel 3

In 18 g einer Mischung aus 200 mmolarem Phosphatpuffer, pH 8,0, und Alkohol (s. Tabelle) suspendierte man 2 g rohe Schweinepankreaslipase. Zu dieser Suspension gab man 1 g eines Copolymerisates (US 3 992 562) aus 100 Teilen N-Vinylpyrrolidon und 2 Teilen N,N'-Divinylethylenharnstoff. Diese Mischung wurde über Nacht in einem zylindrischen Gefäß gerollt. Der beladene Träger wurde abgesaugt und mit einer Lösung versetzt, die 300 mg wäßrige Glutardialdehydlösung (25 %) pro Milliliter der entsprechenden Alkohol-Phosphatpuffer-Lösung enthielt. Nach einer Stunde wurde das Immobilisat abgesaugt und mit der jeweiligen Alkohol-Phosphatpuffer-Lösung gewaschen.

| Alkohol [Gew. -Teile] | | Phosphatpuffer [Gew. -Teile] | Ansatz | $A_{sp}$ [u/g] | $A_{rel.}$ in % |
|---|---|---|---|---|---|
| Methanol 10 | Glyzerin 20 | 70 | 1<br>2 | 950<br>903 | -<br>95 |
| 1,5-Pentandiol 10 | Glyzerin 20 | 70 | 1<br>2 | 895<br>832 | -<br>93 |
| Isopropanol 10 | Glyzerin 20 | 70 | 1<br>2 | 1010<br>950 | -<br>94 |
| Isopropanol - | Glyzerin 30 | 70 | 1<br>2 | 540<br>490 | -<br>91 |

Vergleichsversuch 1 (mit einem anderen Träger)

2 g rohe Schweinepankreaslipase wurden gemäß EP-A-210 499 in 8 g einer Lösung aus 50 Teilen 50 mmolarem Phosphatpuffer $p_H$ 8,0 und 50 Teilen Glyzerin suspendiert und mit 6,5 g Sorbit-$EO_{80}$-epoxid (siehe Beispiel 5) und 4,0 g Polyethyleniminlösung (25 % im Wasser, mit HCl auf $p_H$ 8,0 eingestellt) gemischt. Nach 24 Stunden wurde das entstandene Gel durch ein Sieb mit 1 mm Maschenbreite gedrückt.

| Ansatz | $A_{sp}$ [u/g] | $A_{rel.}$ in % |
|---|---|---|
| 1 | 53 | - |
| 2 | 32 | 60 |
| 3 | 28 | 53 |

Die Immobilisate waren weich und verklebten beim Filtrieren.

Vergleichsversuch 2 (ohne Alkoholzusatz)

In 18 g 200 mmolarem Phosphatpuffer suspendierte man 2 g rohe Schweinepankreaslipase und anschließend 1 g eines Copolymerisats, hergestellt nach US 4 013 825 aus 100 Teilen N-Vinylpyrrolidon und 2 Teilen N,N' -Dinvinylethylenharnstoff. Die Mischung blieb über Nacht stehen und wurde am nächsten Tag abgesaugt und mit 200 mmolarem Phosphatpuffer gewaschen.

Dasselbe Immobilisat wurde zweimal zur Esterspaltung eingesetzt, die spezifischen Aktivitäten stehen in der Tabelle:

| Ansatz | $A_{sp}$ [u/g] | $A_{rel.}$ in % |
|---|---|---|
| 1 | 201 | - |
| 2 | 108 | 54 |

Vergleichsversuch 3

Analog zu Beispiel 1 wurden vier andere Lipasen eingesetzt:

- Lipase Saiken 100 aus Rhizopus japonicus von K.K. Osaka Saihin Kenkyusho
- Lipase M-AP 10 aus Mucor sp. von Amano Pharmaceutical Co. Ltd.
- Piccantase-A aus Mucor miehei von Gist-Brocades
- Lipase My aus Candida cylindracea von Meito Sangyo

Die ersten drei zeigten keine enzymatische Aktivität bei der Esterspaltung, die spezifischen Aktivitäten der Lipase My stehen in der Tabelle:

| Ansatz | $A_{sp}$ (CB) [u/g] | $A_{rel.}$ in % |
|---|---|---|
| 1 | 205 | - |
| 2 | 165 | 80 |

Anwendungsbeispiel

1,4 1 einer 0,05 M auf pH 7 eingestellten Pufferlosung ($Na_2B_4O_7 \cdot 10\ H_2O$, mit konzentrierter Salzsäure eingestellt) wurden auf 6 °C gekühlt. Es wurden 216,1 g (1,5 Mol) razemisches Glycidylbutyrat und 15 g feuchtes, mit pH-7-Pufferlösung gewaschenes Immobilisat zugegeben. Das Gemisch wurde bei 9-10 °C intensiv gerührt und durch Zupumpen von 10 N NaOH (insgesamt 82,5 ml, 0,825 Mol, entsprechend 55 % Hydrolyse) nahe pH 7,3 gehalten. Nach 150 min war die angegebene NaOH-Menge verbraucht. Das Immobilisat wurde abfiltriert, zweimal mit $CH_2Cl_2$, einmal mit pH-8-Puffer gewaschen und anschließend in pH-7-Puffer suspendiert, bei 5-8 °C aufbewahrt. Das Filtrat und die Waschphasen wurden vereinigt, durchgeschüttelt und die organische Phase abgetrennt. Die wäßrige Phase wurde noch dreimal mit $CH_2Cl_2$ extrahiert. Die vereinigten organischen Phasen wurden über Natriumsulfat getrocknet, rotierend i.V. eingeengt und destilliert. Es wurden 73 g R(-)Glycidylbutyrat erhalten (0,5 Mol, 75 % Ausbeute, bezogen auf die bei 55 % Hydrolyse theoretisch erhältlichen 0,67 Mol R(-)Glycidylbutyrat). Drehwert $[\alpha]_{20}^{D}$ = 29,2 °, Kp 83 °/14 mbar.

**Patentansprüche**

**1.** Verfahren zur Herstellung eines Biokatalysators durch Mischen eines Trägers mit einer Lipase und einer wäßrigen Pufferlösung bei pH 5 bis 9 und 0 bis 40 °C, nach 1 Minute bis 3 Tagen Filtrieren und Waschen des Filterkuchens, dadurch gekennzeichnet, daß

a) als Träger ein pulvriges, unlösliches, nur wenig quellbares Copolymerisat mindestens eines N-Vinyllactams mit 4 bis 6 Kohlenstoffatomen mit einem cyclischen Säureamid, das mindestens 2 ethylenisch ungesättigte, copolymerisierbare Gruppen enthält, von denen mindestens eine unmittelbar an Amidstickstoff gebunden ist, und

b) als Lipase rohe Schweinepankreaslipase eingesetzt wird, und

c) der wäßrigen Mischung mindestens ein wasserlöslicher 2- bis 6-wertiger Alkohol mit 2 bis 6 Kohlenstoffatomen zugemischt wird.

2. Verfahren nach Anspruch 1, dadurch gekennzeichnet, daß als N-Vinyllactam N-Vinylpyrrolidon eingesetzt wird.

3. Verfahren nach Anspruch 1 oder 2, dadurch gekennzeichnet, daß als cyclisches Säureamid mit mindestens 2 ethylenisch ungesättigten, copolymerisierbaren Doppelbindungen N,N'-Divinylethylenharnstoff eingesetzt wird.

4. Verfahren nach einem der Ansprüche 1 bis 3, dadurch gekennzeichnet, daß als wasserlöslicher Alkohol mindestens ein mehrwertiger Alkohol oder eine Mischung ein- und mehrwertiger Alkohole eingesetzt wird.

5. Verfahren nach Anspruch 4, dadurch gekennzeichnet, daß als wasserlöslicher Alkohol Glyzerin oder eine Mischung aus Glyzerin und mindestens einer Komponente aus der Gruppe bestehend aus Methanol, Ethanol, Isopropanol und 1,5-Pentandiol eingesetzt wird.

6. Verfahren nach einem der Ansprüche 1 bis 5, dadurch gekennzeichnet, daß der Filterkuchen mit einem Vernetzungsmittel behandelt wird.

7. Verfahren nach Anspruch 6, dadurch gekennzeichnet, daß als Vernetzungsmittel Glutardialdehyd eingesetzt wird.

8. Verfahren nach Anspruch 6, dadurch gekennzeichnet, daß als Vernetzungsmittel ein wasserlöslicher Glycidylether eingesetzt wird, der hergestellt ist durch Anlagerung von 1 bis 30 Äquivalenten eines Epoxids der Formel I

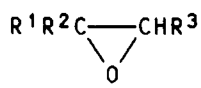

I,

worin die Reste $R^1$, $R^2$ und $R^3$ Wasserstoffatome oder Methyl- oder Ethylgruppen darstellen, pro Äquivalent (bezogen auf OH-Gruppen) eines polyfunktionellen Alkohols mit 2 bis 6 Kohlenstoffatomen oder eines Mono- oder Disaccharids und Umsetzung des so erhaltenen Anlagerungsproduktes mit einem Mol Epichlorhydrin pro Äquivalent OH und anschließende Cyclisierung zum Epoxid.

9. Verfahren nach einem der Ansprüche 1 bis 8, dadurch gekennzeichnet, daß man die Komponenten in folgenden Mengenverhältnissen mischt:
a) 1 bis 50 % des genannten Copolymerisates von N-Vinyllactam,
b) 1 bis 30 % rohe Schweinepankreaslipase,
c) 1 bis 68 % eines oder mehrerer der genannten wasserlöslichen Alkohole,
d) 30 bis 97 % Wasser
sowie den pH-Wert regulierende Zusätze, wobei sich die Prozentangaben jeweils auf das Gewicht der Gesamtmischung beziehen.

10. Biokatalysator, erhältlich nach einem der Ansprüche 1 bis 9.

11. Verwendung des Biokatalysators nach Anspruch 10 zur Razematspaltung von Estern razemischer Alkohole in wasserhaltigem Medium.

12. Verwendung des Biokatalysators nach Anspruch 10 zur Razematspaltung von Glycidylbutyrat.

**Claims**

1. A process for the preparation of a biocatalyst by mixing a substrate with a lipase and an aqueous buffer solution at pH 5-9 and at from 0 to 40 °C, and, after from 1 minute to 3 days, filtering the mixture and washing the filter cake, wherein
a) the substrate used is pulverulent, insoluble, only slightly swellable copolymer of one or more N-vinyllactams of 4 to 6 carbon atoms with a cyclic amide which contains two or more ethylenically

unsaturated, copolymerizable groups, one or more of which are bonded directly to amide nitrogen,

b) the lipase used is crude porcine pancreas lipase and

c) one or more water-soluble dihydric to hexahydric alcohols of 2 to 6 carbon atoms are mixed with the aqueous mixture.

2. A process as claimed in claim 1, wherein the N-vinyllactam used is N-vinylpyrrolidone.

3. A process as claimed in claim 1 or 2, wherein N,N'-divinylethyleneurea is used as the cyclic amide having two or more ethylenically unsaturated, copolymerizable double bonds.

4. A process as claimed in any of claims 1 to 3, wherein one or more polyhydric alcohols or a mixture of a monohydric and a polyhydric alcohol are or is used as the water-soluble alcohol component.

5. A process as claimed in claim 4, wherein a glycerol or a mixture of glycerol and one or more components from the group consisting of methanol, ethanol, isopropanol and 1,5-pentanediol is used as the water-soluble alcohol.

6. A process as claimed in any of claims 1 to 5, wherein the filter cake is treated with a crosslinking agent.

7. A process as claimed in claim 6, wherein the crosslinking agent used is glutardialdehyde.

8. A process as claimed in claim 6, wherein the crosslinking agent used is a water-soluble glycidyl ether which is prepared by an addition reaction of from 1 to 30 equivalents of an epoxide of the formula I

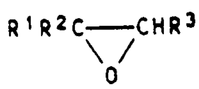

I

where $R^1$, $R^2$ and $R^3$ are each hydrogen, methyl or ethyl, with one equivalent (based on OH groups) of a poly-functional alcohol of 2 to 6 carbon atoms or of a mono-or disaccharide, and reaction of the resulting adduct with one mole of epichlorohydrin per equivalent of OH, followed by cyclization to give the epoxide.

9. A process as claimed in any of claims 1 to 8, wherein the components are mixed in the following proportions:

a) from 1 to 50% of the stated copolymer of N-vinyllactam,

b) from 1 to 30% of crude porcine pancreas lipase,

c) from 1 to 68% of one or more of the stated water-soluble alcohols,

d) from 30 to 97% of water

and the pH-regulating additives, the percentages in each case being based on the weight of the total mixture.

10. A biocatalyst obtainable as claimed in any of claims 1 to 9.

11. Use of a biocatalyst as claimed in claim 10 for resolving a racemate of an ester of a racemic alcohol in an aqueous medium.

12. Use of a biocatalyst as claimed in claim 10 for resolving a racemate of glycidyl butyrate.

**Revendications**

1. Procédé de préparation d'un biocatalyseur par mélange d'un support avec une lipase et une solution tampon aqueuse, à pH 5 à 9 et entre 0 et 40 degrés C, filtration après 1 minute à 3 jours et lavage du tourteau de filtration, caractérisé par le fait que l'on utilise

a) comme support, un copolymérisat pulvérulent peu soluble, seulement peu gonflable, d'au moins un N-vinyllactame ayant 4 à 6 atomes de carbone, avec un amide d'acide cyclique contenant au

moins 2 groupes copolymérisables à insaturation éthylénique, dont l'un au moins est lié directement à de l'azote d'amide, et

b) comme lipase, de la lipase de pancréas de porc brute et

c) que l'on ajoute au mélange aqueux, avec mélange, au moins un alcool bivalent à hexavalent soluble dans l'eau, ayant 2 à 6 atomes de carbone.

**2.** Procédé selon la revendication 1, caractérisé par le fait que l'on utilise, comme N-vinyllactame, de la N-vinylpyrrolidone.

**3.** Procédé selon l'une des revendications 1 ou 2, caractérisé par le fait que l'on utilise de la N,N'-divinyléthylène-urée comme amide d'acide cyclique ayant au moins 2 doubles liaisons copolymérisables à insaturation éthylénique.

**4.** Procédé selon l'une des revendications 1 à 3, caractérisé par le fait que l'on utilise, comme alcool soluble dans l'eau, au moins un polyol ou un mélange d'alcools monovalents et de polyols.

**5.** Procédé selon la revendication 4, caractérisé par le fait que l'on utilise, comme alcool soluble dans l'eau, du glycérol ou un mélange de glycérol et d'au moins un composant choisi dans le groupe constitué de méthanol, éthanol, isopropanol et 1,5-pentanediol.

**6.** Procédé selon l'une des revendications 1 à 5, caractérisé par le fait que le tourteau de filtration est traité avec un agent réticulant.

**7.** Procédé selon la revendication 6, caractérisé par le fait que l'on utilise, comme agent réticulant, du glutardialdéhyde.

**8.** Procédé selon la revendication 6, caractérisé par le fait que l'on utilise, comme agent réticulant, un éther glycidique soluble dans l'eau qui est préparé en fixant par addition 1 à 30 équivalents d'un époxyde de formule I

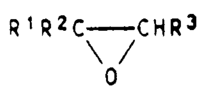

I,

dans laquelle les restes $R^1$, $R^2$ et $R^3$ représentent des atomes d'hydrogène ou des groupes méthyle ou éthyle, par équivalent (rapporté aux groupes OH) d'un polyol à 2 à 6 atomes de carbone, ou d'un mono-ou disaccharide, et réaction du produit d'addition ainsi obtenu avec un mole d'épichlorhydrine par équivalent d'OH, et cyclisation subséquente en époxyde.

**9.** Procédé selon l'une des revendications 1 à 8, caractérisé par le fait que l'on mélange les composants en les proportions en poids suivantes :

a) 1 à 50 % du copolymérisat de N-vinyllactame indiqué plus haut,

b) 1 à 30 % de lipase de pancréas de porc brute,

c) 1 à 68 % d'un ou plusieurs alcools solubles dans l'eau du type cite plus haut,

d) 30 à 97 % d'eau,

ainsi que des additifs réglant la valeur du pH, les pourcentages se rapportant dans chaque cas au poids du mélange total.

**10.** Biocatalyseur obtenu selon l'une des revendications 1 à 9.

**11.** Utilisation du biocatalyseur selon la revendication 10 pour la séparation des racémates d'esters d'alcools racémiques en milieu contenant de l'eau.

**12.** Utilisation du biocatalyseur selon la revendication 10 pour la séparation des racémates du butyrate de glycidyle.